# EUROPÄISCHE PATENTANMELDUNG

(11) **EP 0 695 735 A1**
(43) Veröffentlichungstag der Anmeldung: **07.02.1996**
(21) Anmeldenummer: 95111594.8
(22) Anmeldetag: 24.07.1995
(51) Int. Cl.: C07C 53/48, C07C 51/62, C07C 51/58

(54) **Verfahren zur Herstellung von Trichloracetylchlorid**

(30) Priorität: 02.08.1994 DE 4427303
(71) Anmelder: HOECHST AKTIENGESELLSCHAFT, D-65929 Frankfurt am Main (DE)
(72) Erfinder: Ebmeyer, Frank, Dr., D-86153 Augsburg (DE); Metzenthin, Tobias, Dr., D-65929 Frankfurt (DE); Siegemund, Günter, Dr., D-65719 Hofheim (DE)

(57) **Zusammenfassung**

Die Erfindung betrifft ein Verfahren zur Herstellung von Trichloracetylchlorid durch Reaktion von Acetylchlorid oder Acetaldehyd oder deren chlorierten Derivaten mit Chlor in Gegenwart von Aktivkohle als Katalysator.

## Beschreibung

Trichloracetylchlorid ist ein wichtiges Ausgangsprodukt für die Herstellung von Pharma- und Pflanzenschutzwirkstoffen.

Nach US-A-3 751 461 wird Trichloracetylchlorid durch Chlorierung von Acetylchlorid, das teilweise chloriert sein kann, in der Flüssigphase in Gegenwart von organischen Stickstoffbasen als homogenen Katalysatoren erhalten. Dieses Verfahren erfordert einen diskontinuierlichen Reaktorbetrieb. Außerdem ist eine destillative Abtrennung des Reaktionsproduktes von dem darin gelösten Katalysator notwendig.

Es wurde nun überraschend gefunden, daß Trichloracetylchlorid mit Hilfe von Aktivkohle, also einem heterogenen und daher leicht abtrennbaren Katalysator, durch Chlorierung von Acetylchlorid oder Acetaldehyd oder deren chlorierten Derivaten hergestellt werden kann.

Gegenstand der Erfindung ist ein Verfahren zur Herstellung von Trichloracetylchlorid durch katalytische Reaktion von Acetylchlorid oder Acetaldehyd, die chloriert sein können, mit Chlor, dadurch gekennzeichnet, daß man Aktivkohle als Katalysator verwendet.

Das Verfahren wird vorzugsweise nach Art üblicher kontinuierlicher Gasreaktionen an Festbettkatalysatoren durchgeführt, indem man das Gasgemisch aus organischem Ausgangsmaterial und Chlor, ggf. mit Stickstoff verdünnt, durch ein beheiztes Reaktionsrohr leitet, das mit Aktivkohle gefüllt ist.

Das Reaktionsrohr wird vorzugsweise vertikal aufgestellt und besteht aus einem gegen die Reaktionspartner hinreichend resistenten Werkstoff.

Das Verfahren findet im allgemeinen bei Drücken von 10⁻¹ bis 10 bar statt, vorzugsweise bei 1 bis 7 bar, insbesondere bei 1 bis 5 bar.

Das Molverhältnis von Chlor zum organischen Ausgangsmaterial wird im allgemeinen so geregelt, daß 1 bis 10, vorzugsweise 1 bis 4, Chlormoleküle auf jedes Wasserstoffatom im Ausgangsmaterial entfallen.

Die Reaktion wird im allgemeinen bei Temperaturen von 20 bis 300°C, bevorzugt bei 100 bis 250°C, insbesondere bei 120 bis 200°C durchgeführt. Eine gleichmäßige Temperaturverteilung kann dadurch erreicht werden, daß das Reaktionsrohr mit einer wärmeübertragenden Flüssigkeit umgeben ist.

Der Umsatz des eingesetzten organischen Ausgangsmaterials ist im allgemeinen vollständig.

Das bei der Umsetzung entstehende und Trichloracetylchlorid, Chlorwasserstoff und etwas Chlor enthaltende Gas wird kondensiert und destillativ aufgearbeitet.

Die Vorteile liegen in der einfachen Durchführung mit einem heterogenen Katalysator bei sehr hohem Umsatz an organischer Ausgangsverbindung.

Das organische Ausgangsmaterial kann auch in flüssiger Form in Gegenwart von Chlor über ein fixiertes Katalysatorbett geleitet werden; dies kann in kontinuierlichem oder diskontinuierlichem Reaktorbetrieb realisiert werden. Dabei arbeitet man im allgemeinen bei Temperaturen von 20 bis 200°C, vorzugsweise 80 bis 120°C, sowie Drücken von 1 bis 10 bar, vorzugsweise 1 bis 7 bar.

Das erfindungsgemäße Verfahren wird durch die nachfolgenden Beispiele näher erläutert. Wenn nichts anderes angegeben wird, erreichte der Umsatz 100 %. Die Prozentangaben sind Molprozente, soweit nichts anderes gesagt wird.

### Beispiele

Es wurde ein Rohr aus V4A-Stahl mit einem Innendurchmesser von 5,0 cm und einer Länge von 70 cm verwendet, welches elektrisch beheizt wurde. Die Innentemperatur des Reaktors wurde mit Hilfe eines axialen Thermoelements in einem Gehäuse bestimmt. Der vertikal installierte Reaktor wurde mit 1,0 l (450 g) handelsüblicher Aktivkohle (Granulat) beschickt, welche in einem Stickstoffstrom langsam aufgeheizt und so lange auf 300°C erwärmt wurde,bis sie kein Wasser mehr abgab. Anschließend wurde bei 200°C ein Strom von 40 g/h Chlor über die Katalysatorschüttung geleitet, wobei sich ein um 40°C heißerer "Hot-Spot" ausbildete, der allmählich durch die Katalysatorschüttung wanderte. Ein so vorbehandelter Katalysator wurde in allen Beispielen verwendet.

### Beispiel 1

Die Reaktionspartner Chlor (Reinheit > Gew. 99 %) und Dichloracetylchlorid (Reinheit > Gew. 99 %) wurden in einen dem Reaktor vorgeschalteten, auf 140°C beheizten Verdampfer eindosiert. Hier erfolgte die Verdampfung der organischen Komponente und die Durchmischung mit dem gasförmigen Chlor. Anschließend wurden die Komponenten gasförmig in den auf eine Innentemperatur von ca. 195 °C beheizten und mit dem vorbehandelten Katalysator gefüllten Reaktor geführt. Bei einer Einspeisung von 48 g/h Dichloracetylchlorid und 41 g/h Chlor ergab die Analyse der hinter dem Reaktorausgang kondensierten Flüssigkeit:

| | |
|---|---|
| Trichloracetylchlorid: | 98,5 % |
| Dichloracetylchlorid: | 1,0 % |
| Tetrachlorkohlenstoff: | 0,5 % |

### Beispiel 2

In die oben beschriebene Reaktionsapparatur wurde Chlor und Dichloracetylchlorid eindosiert. Die Temperaturverteilung innerhalb des Reaktors betrug 200°C am heißesten und 180°C am kältesten Punkt der Katalysatorschüttung. Bei einer Dosierung von 75 g/h Dichloracetylchlorid und 63 g/h Chlor ergab die regelmäßige Analyse der hinter dem Reaktorausgang kondensierten Flüssigkeit:

| | |
|---|---|
| Trichloracetylchlorid: | 98,0 % |
| Dichloracetylchlorid: | 1,5 % |
| Tetrachlorkohlenstoff: | 0,5 % |

### Beispiel 3

In den oben beschriebenen Reaktor dosierte man 24 g/h Acetylchlorid und 79 g/h Chlor. Die beiden Reaktionspartner wurden in den auf 60°C vorgeheizten Verdampfer eindosiert. Die Temperaturverteilung innerhalb des Reaktors betrug 225°C am heißesten und 190°C am kältesten Punkt der Katalysatorschüttung. Die regelmäßige Analyse der während einer Betriebszeit von 5 h hinter dem Reaktor kondensierten Flüssigkeit ergab die Zusammensetzung:

| | |
|---|---|
| Trichloracetylchlorid: | 96,6 % |
| Dichloracetylchlorid: | 1,0 % |
| Tetrachlorkohlenstoff: | 2,4 % |

### Beispiel 4

In den oben beschriebenen Reaktor dosierte man 16 g/h Acetaldehyd und 130 g/h Chlor über den auf 60°C beheizten Verdampfer ein. Bei einer Temperaturverteilung zwischen 250°C (Maximum) und 200°C (Minimum) ergab die Umsetzung eine Produktflüssigkeit der Zusammensetzung:

| | |
|---|---|
| Trichloracetylchlorid: | 89,9 % |
| Tetrachlorkohlenstoff: | 5,5 % |
| Chloroform: | 5,6 % |

### Beispiel 5

In den oben beschriebenen Reaktor dosierte man 39 g/h Trichloracetaldehyd und 30 g/h Chlor über den auf 115°C beheizten Verdampfer ein. Bei einer Temperaturverteilung zwischen 250°C (Maximum) und 230°C (Minimum) ergab die Umsetzung eine Produktflüssigkeit der Zusammensetzung:

| | |
|---|---|
| Trichloracetylchlorid: | 97,3 % |
| Trichloracetaldehyd: | 1,0 % |
| Tetrachlorkohlenstoff: | 1,7 % |

### Beispiel 6

In einem 500 ml Glaskolben legt man 17 g getrocknete Aktivkohle (Granulat) vor, versetzt vorsichtig mit 100 g Dichloracetylchlorid und erhitzt anschließend unter Rühren auf 110°C. Anschließend leitet man im Verlauf von 3,5 bis 4 Stunden 88 g gasförmiges Chlor in die Reaktionsmischung ein. Der Stand der Chlorierung läßt sich gaschromatographisch verfolgen. Nach 4,5 Stunden läßt man den Reaktionsansatz abkühlen und vertreibt überschüssiges Chlor durch Einleiten von trockener Luft bzw. Stickstoff. Anschließend filtriert man von der Aktivkohle ab und erhält 120 g Trichloracetylchlorid mit einer Reinheit von 96 %.

## Patentansprüche

1. Verfahren zur Herstellung von Trichloracetylchlorid durch katalytische Reaktion von Acetylchlorid oder Acetaldehyd, die chloriert sein können, mit Chlor, dadurch gekennzeichnet, daß man Aktivkohle als Katalysator verwendet.

2. Verfahren nach Anspruch 1, dadurch gekennzeichnet, daß man Chloracetylchlorid als Ausgangsmaterial verwendet.

3. Verfahren nach Anspruch 1, dadurch gekennzeichnet, daß man Dichloracetylchlorid als Ausgangsmaterial verwendet.

4. Verfahren nach Anspruch 1, dadurch gekennzeichnet, daß man Chloracetaldehyd als Ausgangsmaterial verwendet.

5. Verfahren nach Anspruch 1, dadurch gekennzeichnet, daß man Dichloracetaldehyd als Ausgangsmaterial verwendet.

6. Verfahren nach Anspruch 1, dadurch gekennzeichnet, daß man Trichloracetaldehyd als Ausgangsmaterial verwendet.

7. Verfahren nach einem der Ansprüche 1 bis 6, dadurch gekennzeichnet, daß man die Reaktion kontinuierlich in der Gasphase bei 100 bis 250°C durchführt.

8. Verfahren nach einem der Ansprüche 1 bis 7, dadurch gekennzeichnet, daß das Molverhältnis von Chlor zu organischem Ausgangsmaterial so gewählt wird, daß 1 bis 4 Chlormoleküle auf jedes Wasserstoffatom des Ausgangsmaterials entfallen.
